(19) Europäisches Patentamt
European Patent Office
Office européen des brevets

(11) Veröffentlichungsnummer : **0 008 370**
**B1**

(12) # EUROPÄISCHE PATENTSCHRIFT

(45) Veröffentlichungstag der Patentschrift : **02.12.81**

(51) Int. Cl.³ : **C 07 C 83/10**, C 07 D307/68

(21) Anmeldenummer : **79102534.9**

(22) Anmeldetag : **18.07.79**

(54) **N-Cyclohexyl-N-methoxyacetoacetamid, Verfahren zu seiner Herstellung und seine Verwendung zur Herstellung von O-Methyl-N-cyclohexyl-2,5-dimethylfuran-3-hydroxamsäure.**

(30) Priorität : **21.07.78 DE 2832102**

(43) Veröffentlichungstag der Anmeldung : **05.03.80 (Patentblatt 80/05)**

(45) Bekanntmachung des Hinweises auf die Patenterteilung : **02.12.81 Patentblatt 81/48**

(84) Benannte Vertragsstaaten : **AT BE CH DE FR GB IT NL SE**

(56) Entgegenhaltungen :
**DE - A - 1 931 386**
**DE - A - 2 227 547**

(73) Patentinhaber : **BASF Aktiengesellschaft**
**Carl-Bosch-Strasse 38**
**D-6700 Ludwigshafen (DE)**

(72) Erfinder : **Linhart, Friedrich, Dr. Dipl.-Chem.**
**Leisberg 61**
**D-6900 Heidelberg (DE)**
Erfinder : **Girgensohn, Bjoern, Dr. Dipl.-Chem.**
**Neckarpromenade 38**
**D-6800 Mannheim 1 (DE)**
Erfinder : **Zeeh, Bernd, Dr. Dipl.-Chem.**
**Thorwaldsenstrasse 5**
**D-6700 Ludwigshafen (DE)**

**EP 0 008 370 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

N-Cyclohexyl-N-methoxyacetoacetamid, Verfahren zu seiner Herstellung und seine Verwendung zur Herstellung von O-Methyl-N-cyclohexyl-2,5-dimethylfuran-3-hydroxamsäure

Die Erfindung betrifft N-Cyclohexyl-N-methoxyacetoacetamid, ein Verfahren zu seiner Herstellung und dessen Verwendung als Vorprodukt zur Herstellung von O-Methyl-N-cyclohexyl-2,5-dimethylfuran-3-hydroxamsäure.

Für die Herstellung von Furanderivaten gibt es mehrere Möglichkeiten, die dem Fachmann geläufig sind. So ist beispielsweise bekannt, O-Methyl-N-cyclohexyl-2,5-dimethylfuran-3-hydroxamsäure aus 2,5-Dimethylfuran-3-carbonsäurechlorid durch Umsetzung mit N-Cyclohexylhydroxylamin und anschließende Methylierung des erhaltenen Zwischenproduktes mit Dimethylsulfat herzustellen (DE-A 24 55 082). Diese Arbeitsweise ist jedoch aufwendig und nicht voll befriedigend, insbesondere wegen der schweren Zugänglichkeit des 2,5-Dimethylfuran-3-carbonsäurechlorids und der Entstehung von Nebenprodukten.

Es wurde nun ein Weg gefunden, der sich zur Herstellung von O-Methyl-N-cyclohexyl-2,5-dimethylfuran-3-hydroxamsäure, einem bedeutsamen Fungizid, vorzüglich eignet. Ausgangsprodukt für das neue Herstellungsverfahren ist N-Cyclohexyl-N-methoxyacetoacetamid. Dieses wird unter Verwendung eines sauren Katalysators mit einem α-Acyloxypropionaldehyd nach folgendem Reaktionsschema kondensiert :

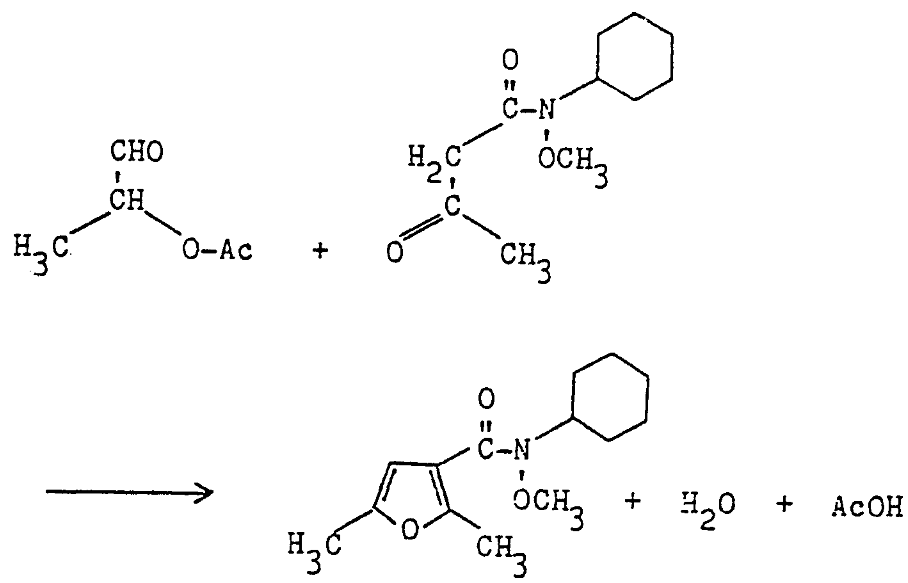

Das N-Cyclohexyl-N-methoxyacetoacetamid ist neu. Diese Verbindung, ihre Herstellung und ihre Verwendung zu Synthese des genannten Furanderivates sind Gegenstand dieser Erfindung. Man erhält die Verbindung ohne Schwierigkeiten, wenn man O-Methyl-N-cyclohexylhydroxylamin in Anwesenheit oder Abwesenheit eines Lösungs- oder Verdünnungsmittels bei Temperaturen zwischen 0 und 80 °C, vorzugsweise zwischen 30 und 50 °C, mit Diketen umsetzt. Als Lösungs- oder Verdünnungsmittel eignen sich solche, die unter den Reaktionsbedingungen inert sind, z.B. Kohlenwasserstoffe und chlorierte Kohlenwasserstoffe, wie Cyclohexan, Benzine, Toluol, Xylole, Chlorbenzole, höhere Aromaten, Ether, wie Tetrahydrofuran, Dioxan, Ester, wie Essigester, Amide, wie Dimethylformamid, chlorierte Kohlenstoffe und andere oder Gemische solcher Flüssigkeiten. Es können aber auch Flüssigkeiten benutzt werden, die mit Diketen langsamer reagieren als O-Methy-N-cyclohexylamin, z.B. Wasser. Man kann auch das Reaktionsprodukt als Lösungs- und Verdünnungsmittel benutzen.

Man läßt die beiden Komponenten zweckmäßigerweise im äquimolaren Verhältnis aufeinander einwirken, da die Umsetzung praktisch vollständig verläuft und es somit nicht erforderlich ist, einen der überschüssigen Reaktionspartner aus dem Reaktionsansatz zurückzugewinnen.

Das für die Herstellung von N-Cyclohexyl-N-methoxyacetoacetamid verwendete O-Methyl-N-cyclohexylhydroxylamin ist in Form seines Hydrochlorids bekannt (K. G. TAYLOR, S. R. ISAAC, M. S. CLARK, J. org. Chem. *41,* 1 135-40 (1976)) und aus diesem leicht mit Hilfe einer Base in Freiheit zu setzen.

Die Verwendung des N-Cyclohexyl-N-methoxyacetoacetamid zur Herstellung von O-Methyl-N-cyclohexyl-2,5-dimethylfuran-3-hydroxamsäure geschieht durch Umsetzen mit α-Acetoxypropionaldehyd in einem unter den Reaktionsbedingungen inerten Lösungsmittel bei Temperaturen zwischen 20 und 80 °C, vorzugsweise zwischen 40 und 55 °C. Hierbei ist est wesentlich, daß das bei der Reaktion entstehende Wasser aus dem Reaktionsgemisch entfernt wird, sei es durch azeotrope Destillation mit einem Lösungsmittel, z.B. Methylenchlorid, sei es dadurch, daß es durch ein Trockenmittel, z.B. Calciumchlorid, Magnesiumsulfat, Calciumsulfat, Kieselgel, Molekularsiebe, oder vorteilhafterweise durch den Katalysator, z.B. Eisen-III-chlorid, gebunden wird. In diesem Fall sind vom Katalysator größere Mengen

einzusetzen. Nach dem Zersetzen des Reaktionsgemisches mit Wasser und Abtrennen der organischen Phase erhält man, gegebenenfalls nach Entfernen des Lösungsmittels, die O-Methyl-N-cyclohexyl-2,5-dimethylfuran-3-hydroxamsäure in sehr guter Ausbeute und befriedigender Reinheit. Dieses Ergebnis war nach der DE-AS 22 27 547 nicht zu erwarten, da bei dem darin offenbarten Verfahren Acetoacetamide verwendet werden, die ausdrücklich keinen O-Substituenten am Amidstickstoff tragen.

Inerte Lösungsmittel, in denen die Umsetzung zu den Heterocyclen vorgenommen werden kann, sind beispielsweise Alkohole, wie Isobutanol, Methanol, Ethanol, Propanol, Isopropanol und höhere Alkohole, Halogenkohlenwasserstoffe, wie Methylenchlorid, Dichlormethan, Ester, wie Essigester, Ether, wie Tetrahydrofuran, Dioxan, Methylglykol, aromatische Verbindungen, wie Benzol, Toluol, Xylol oder z.B. Cyclohexan.

Als saure Katalysatoren eignen sich Protonensäuren und Lewissäuren, z.B. p-Toluolsulfonsäure, Schwefelsäure, Phosphorsäure, Polyphosphorsäuren, oder beispielsweise Eisen-III-chlorid, Zinkchlorid, Zinn-IV-chlorid, Brotrifluorid, Aluminiumchlorid.

Man kann drucklos oder unter erhöhtem Druck arbeiten, bis ungefähr 700 atü, gewünschtenfalls auch in einer Inertgasatmosphäre.

Als Acylreste im α-Acyloxypropionaldehyd sind solche mit 1 bis 4 Kohlenstoffatomen bevorzugt, wie Formyl, Acetyl, Propionyl, Butyryl.

Der α-Acyloxypropionaldehyd ist bekannt und in bekannter Weise durch Hydroformylierung des entsprechenden Vinylesters erhältlich.

Die folgenden Ausführungsbeispiele erläutern die Erfindung. Unter Teilen sind Gewichtsteile zu verstehen.

### Beispiel 1

N-Cyclohexyl-N-methoxyacetoacetamid :

Zu einer Lösung von 129 Teilen O-Methyl-N-cyclohexylhydroxylamin in 400 Teilen Cyclohexan tropft man bei 35 bis 45 °C 84 Teile Diketen. Zur vollständigen Umsetzung rührt man 45 Minuten bei 45 °C nach. Nach Abziehen des Lösungsmittels unter vermindertem Druck erhält man 213 Teile N-Cyclohexyl-N-methoxyacetoacetamid, das unter einem Druck von $4 \cdot 10^{-4}$ bar (0,3 Torr) bei 113 bis 117 °C siedet.

### Beispiel 2

O-Methyl-N-cyclohexyl-2,5-dimethylfuran-3-hydroxamsäure :

Zu einer Suspension von 19,4 Teilen Eisen-III-chlorid in 100 Teilen Isobutanol tropft man bei 40 bis 45 °C ein Gemisch aus 85,2 Teilen N-Cyclohexyl-N-methoxyacetoacetamid und 58,2 Teilen 95,7 %-igem α-Acetoxypropionaldehyd. Anschließend hält man 7,5 Stunden bei 45 °C. Man versetzt das Reaktionsgemisch mit Wasser, extrahiert mit Methylenchlorid und dampft den Extrakt ein. Man erhält 104 Teile Rohprodukt, das sich unter einem Druck von $4 \cdot 10^{-4}$ bar (0,3 Torr) bei 138 bis 145 °C destillieren läßt, wobei man 88,5 Teile reine O-Methyl-N-cyclohexyl-2,5-dimethylfuran-3-hydroxamsäure erhält.

### Beispiel 3

O-Methyl-N-cyclohexyl-2,5-dimethylfuran-3-hydroxamsäure :

Zu 7,6 Teilen p-Toluolsulfonsäure in 320 Teilen Dichlormethan gibt man innerhalb von 20 Minuten ein Gemisch aus 85,2 Teilen N-Cyclohexyl-N-methoxyacetoacetamid und 58,8 Teilen 94,7 %-igem α-Acetoxypropionaldehyd. Anschließend wir 7,5 Stunden lang mit Hilfe eines Wasserabscheiders unter Rückfluß Wasser ausgekreist. Die abgekühlte Reaktionslösung wird 3-mal mit je 100 Teilen Wasser ausgeschüttet, getrocknet und eingedampft. Bei der Destillation der erhaltenen 103,2 Teile Rohprodukt erhält man 90,6 Teile reine O-Methyl-N-cyclohexyl-2,5-dimethylfuran-3-hydroxamsäure.

**Ansprüche** (für die Vertragsstaaten BE, CH, DE, FR, GB, IT, NL, SE)

1. N-Cyclohexyl-N-methoxyacetoacetamid.

2. Verfahren zur Herstellung von N-Cyclohexyl-N-methoxyacetoacetamid, dadurch gekennzeichnet, daß man O-Methyl-N-cyclohexylhydroxylamin bei Temperaturen zwischen 0 und 80 °C mit Diketen umsetzt.

3. Verwendung von N-Cyclohexyl-N-methoxyacetoacetamid zur Herstellung von O-Methyl-N-cyclohexyl-2,5-dimethylfuran-3-hydroxamsäure, dadurch gekennzeichnet, daß man N-Cyclohexyl-N-methoxyacetoacetamid bei Temperaturen zwischen 20 und 80 °C in Gegenwart eines sauren katalysators mit einem α-Acyloxypropionaldehyd umsetzt und gleichzeitig das Reaktionswasser bindet oder aus dem Reaktionsgemisch entfernt.

4. Verwendung gemäß Anspruch 3, dadurch gekennzeichnet, daß man als Katalysator und wasserbindendes Mittel Eisen-III-chlorid benutzt.

5. Verwendung gemäß Anspruch 3, dadurch gekennzeichnet, daß man als Katalysator p-Toluolsulfonsäure benutzt und das entstehende Reaktionswasser azeotrop abdestilliert.

6. Verwendung gemäß Anspruch 5, dadurch gekennzeichnet, daß man Dichlormethan als Schleppmittel für die azeotrope Wasserentfernung benutzt.

7. Verwendung gemäß Anspruch 3, dadurch gekennzeichnet, daß man als α-Acyloxypropionaldehyd den α-Acetoxypropionaldehyd benutzt.

**Ansprüche** (für den Vertragsstaat AT)

1. Verfahren zur Herstellung von N-Cyclohexyl-N-methoxyacetoacetamid, dadurch gekennzeichnet, daß man O-Methyl-N-cyclohexylhydroxylamin bei Temperaturen zwischen 0 und 80 °C mit Diketen umsetzt.

2. Verwendung von N-Cyclohexyl-N-methoxyacetoacetamid zur Herstellung von O-Methyl-N-cyclohexyl-2,5-dimethylfuran-3-hydroxamsäure, dadurch gekennzeichnet, daß man N-Cyclohexyl-N-methoxyacetoacetamid bei Temperaturen zwischen 20 und 80 °C in Gegenwart eines sauren Katalysators mit einem α-Acyloxypropionaldehyd umsetzt und gleichzeitig das Reaktionswasser bindet oder aus dem Reaktionsgemisch entfernt.

3. Verwendung gemäß Anspruch 2, dadurch gekennzeichnet, daß man als katalysator und wasserbindendes Mittel Eisen-III-chlorid benutzt.

4. Verwendung gemäß Anspruch 2, dadurch gekennzeichnet, daß man als Katalysator p-Toluolsulfonsäure benutzt und das entstehende Reaktionswasser azeotrop abdestilliert.

5. Verwendung gemäß Anspruch 4, dadurch gekennzeichnet, daß man Dichlormethan als Schleppmittel für die azeotrope Wasserentfernung benutzt.

6. Verwendung gemäß Anspruch 2, dadurch gekennzeichnet, daß man als α-Acyloxypropionaldehyd den α-Acetoxypropionaldehyd benutzt.

**Claims** (for the Contracting states : BE, CH, DE, FR, GB, IT, NL, SE)

1. N-Cyclohexyl-N-methoxyacetoacetamide.

2. A process for the preparation of N-cyclohexyl-N-methoxyacetoacetamide, characterized in that O-methyl-N-cyclohexylhydroxylamine is reacted with diketene at from 0 to 80 °C.

3. Use of N-cyclohexyl-N-methoxyacetoacetamide for the preparation of O-methyl-N-cyclohexyl-2,5-dimethylfuran-3-hydroxamic acid, characterized in that N-cyclohexyl-N-methoxyacetoacetamide is reacted with an α-acyloxypropionaldehyde at from 20 to 80 °C in the presence of an acid catalyst, and at the same time the water of reaction is bound or removed from the reaction mixture.

4. Use according to claim 3, characterized in that iron-III chloride is used as the catalyst and water-binding agent.

5. Use according to claim 3, characterized in that p-toluenesulfonic acid is used as the catalyst, and the water of reaction which is formed is distilled off azeotropically.

6. Use according to claim 5, characterized in that methylene chloride is employed as the entraining agent for the azeotropic removal of water.

7. Use according to claim 3, characterized in that α-acetoxypropionaldehyde is used as the α-acyloxypropionaldehyde.

**Claims** (for the Contracting state AT)

1. A process for the preparation of N-cyclohexyl-N-methoxyacetoacetamide, characterized in that O-methyl-N-cyclohexylhydroxylamine is reacted with diketene at from 0 to 80 °C.

2. Use of n-cyclohexyl-N-methoxyacetoacetamide for the preparation of O-methyl-N-cyclohexyl-2,5-dimethylfuran-3-hydroxamic acid, characterized in that N-cyclohexyl-N-methoxyacetoacetamide is reacted with an α-acyloxypropionaldehyde at from 20 to 80 °C in the presence of an acid catalyst, and at the same time the water of reaction is bound or removed from the reaction mixture.

3. Use according to claim 2, characterized in that iron-III chloride is used as the catalyst and water-binding agent.

4. Use according to claim 2, characterized in that p-toluenesulfonic acid is used as the catalyst, and the water of reaction which is formed is distilled off azeotropically.

5. Use according to claim 4, characterized in that methylene chloride is employed as the entraining agent for the azeotropic removal of water.

6. Use according to claim 2, characterized in that α-acetoxypropionaldehyde is used as the α-acyloxypropionaldehyde.

**Revendications** (pour les Etats contractants BE, CH, DE, FR, GB, IT, NL, SE)

1. Le N-cyclohexyl-N-méthoxyacétoacétamide.

2. Procédé de préparation du N-cyclohexyl-N-méthoxyacétoacétamide, caractérisé en ce que l'on fait réagir la O-méthyl-N-cyclohexyl-hydroxylamine à des températures de 0 à 80 °C avec du dicétène.

3. Utilisation du N-cyclohexyl-N-méthoxyacétoacétamide pour la préparation de l'acide O-méthyl-N-cyclohexyl-2,5-diméthyl-furanne-3-hydroxamique, caractérisée en ce que l'on fait réagir le N-cyclohexyl-N-méthoxyacétoacétamide à des températures entre 20 et 80 °C, en présence d'un catalyseur acide, avec un aldéhyde α-acyloxypropionique avec fixation simultanée de l'eau de réaction ou élimination de celle-ci du mélange réactionnel.

4. Utilisation suivant la revendication 3, caractérisée en ce que le chlorure ferrique sert à la fois de catalyseur et de fixateur de l'eau.

5. Utilisation suivant la revendication 3, caractérisée en ce que le catalyseur est l'acide paratoluène sulfonique et l'eau de réaction qui se forme est séparée par distillation azéotrope.

6. Utilisation suivant la revendication 5, caractérisée en ce que l'on emploie le dichlorométhane comme agent d'entraînement pour l'élimination azéotrope de l'eau.

7. Utilisation suivant la revendication 3, caractérisée en ce que l'aldéhyde α-acyloxypropionique employé est l'aldéhyde α-acétoxypropionique.

**Revendications** (pour l'Etat contractant AT)

1. Procédé de préparation du N-cyclohexyl-N-méthoxyacétoacétamide, caractérisé en ce que l'on fait réagir la O-méthyl-N-cyclohexyl-hydroxylamine à des températures de 0 à 80 °C avec du dicétène.

2. Utilisation du N-cyclohexyl-N-méthoxyacétoacétamide pour la préparation de l'acide O-méthyl-N-cyclohexyl-2,5-diméthyl-furanne-3-hydroxamique, caractérisée en ce que l'on fait réagir le N-cyclohexyl-N-méthoxyacétoacétamide à des températures entre 20 et 80 °C, en présence d'un catalyseur acide, avec un aldéhyde α-acyloxypropionique avec fixation simultanée de l'eau de réaction ou élimination de celle-ci du mélange réactionnel.

3. Utilisation suivant la revendication 2, caractérisée en ce que le chlorure ferrique sert à la fois de catalyseur et de fixateur de l'eau.

4. Utilisation suivant la revendication 2, caractérisée en ce que le catalyseur est l'acide paratoluène sulfonique et l'eau de réaction qui se forme est séparée par distillation azéotrope.

5. Utilisation suivant la revendication 4, caractérisée en ce que l'on emploie le dichlorométhane comme agent d'entraînement pour l'élimination azéotrope de l'eau.

6. Utilisation suivant la revendication 2, caractérisée en ce que l'aldéhyde α-acyloxypropionique employé est l'aldéhyde α-acétoxypropionique.